# EUROPEAN PATENT APPLICATION

(11) **EP 4 513 501 A1**
(43) Date of publication of application: **26.02.2025**
(21) Application number: 23192440.8
(22) Date of filing: 21.08.2023
(51) Int. Cl.: G16H 10/60, G06F 16/34, G16H 15/00, G16H 30/40

(54) **METHODS AND SYSTEMS FOR PROVIDING A TEXT SUMMARY FOR A MEDICAL IMAGE DATA SET**

(71) Applicant: Siemens Healthineers AG, 91301 Forchheim (DE)
(72) Inventor: Kohle, Sven, 91056 Erlangen (DE); Speier, Christoph, 91052 Erlangen (DE)
(74) Representative: Siemens Healthineers Patent Attorneys

(57) **Abstract**

Systems and computer-implemented methods are provided for providing a text summary (TS) for a medical image data set (MIDS). Specifically, the following steps are proposed:
- receiving (S10) the medical image data set (MIDS) of a patient,
- identifying (S20) at least one compartment (ORG) in the medical image data set (MIDS),
- accessing (S30) supplementary information (SI) associated to the patient in a medical information system (40),
- providing (S40) a text generation function (LLM) configured to provide, for a specific compartment, a natural language text summary (TS) summarizing medical information pertaining to the specific compartment,
- applying (S50) the text generation function (LLM) to the supplementary information (SI) so as to generate a text summary (TS) for the at least one compartment (ORG), and
- providing (S60) the text summary (TS) to a user (U) via a user interface (10).

## Description

Embodiments of the invention concern systems and methods in the field of automatically or semi-automatically generating natural language text. Specifically, embodiments of the invention concern systems and methods for providing a text summary for a medical image data set. More specifically, embodiments of the invention concern system and methods for summarizing findings for individual compartments such as organs or anatomies visible in the medical image data set in natural language text.

In order to create a sound medical report and to produce the right diagnosis, findings, and conclusions, radiologists need to integrate a lot of information from diverse sources. Typically, radiologists must perform several tasks in parallel when evaluating medical image data and preparing a report. Primarily, they must analyze the medical images and summarize their observations and impressions in a radiological report. In addition, they must consider additional information about the patient. This information can, for example, come from images of different modalities or measurement protocols, as well as from information in the patient's health record, laboratory findings, previous images, etc. Thus, the types and number of the individual reporting tasks to be undertaken depends on the available interdisciplinary data, i.e., medical images and other available patient specific medical data.

What is more, a good amount of information is hidden in prosaic text (previous reports, physician's notes, lab reports etc.). Reading and understanding this information in its entirety can require a major amount of time and slows down the radiologists. Therefore, some radiologists do not even engage in reading previous reports and other documentation of the patient. In turn, this may reduce the quality of the radiological examination work product.

To improve on this aspect, it has been proposed to automatically access and filter the available information of a patient and present this information to the radiologist. One issue is that it is exceedingly difficult for the radiologist to understand why a certain piece of information was retrieved. This causes insecurity at the part of the radiologists as to whether or not the information is relevant and complete.

This is even more so if the information is provided by an AI-module or AI-Chatbot of the likes of ChatGPT and other large language models. Often these algorithms are perceived as a black box. In addition, every case is different and information relevant for one case may be irrelevant for another case simply because the image data the radiologist must analyze shows different anatomies.

Despite of the impressive performances of large language models, methods and systems are still at large which allow for the dedicated retrieval of information tailored to the medical image data set under review.

Against this background it is an object of embodiments of the present invention to provide systems and methods for processing the available data of a patient so as to retrieve and further process supplementary information and turn this information into actionable results which optimally complement a medical image data set and/or the performance of a diagnostic task based on the medical image data set.

In the following, the technical solution according to the present invention is described with respect to the claimed systems as well as with respect to the claimed methods. Features, advantages, or alternative embodiments described herein can likewise be assigned to other claimed objects and vice versa. In other words, claims addressing the inventive method can be improved by features described or claimed with respect to the systems. In this case, e.g., functional features of the methods are embodied by objective units or elements of the systems.

Features and alternate forms of embodiments of data structures and/or functions for methods and systems for providing can be transferred to analogous data structures and/or functions for methods and systems for providing trained functions. Analogous data structures can, in particular, be identified by using the prefix "training". Furthermore, the trained functions used in methods and system for providing a text summary can, in particular, have been adjusted and/or trained and/or provided by methods and systems for adjustment of trained functions.

According to an aspect, a computer-implemented method for providing a text summary for a medical image data set is provided. The method comprises a plurality of steps. One step is directed to receive the medical image data set of a patient. Another step is directed to identify at least one compartment in the medical image data set. Another step is directed to access < information associated to the patient in a medical information system. Another step is directed to provide a text generation function configured to provide, for a specific compartment, a natural language text summary summarizing medical information pertaining to the specific compartment. Another step is directed to apply the text generation function to the supplementary information so as to generate a text summary for the at least one compartment. Another step is directed to provide the text summary to a user via a user interface.

The medical image data set may be a two-dimensional image. Further, the medical image data set may be a three-dimensional image. Further, the medical image may be a four-dimensional image, where there are three spatial and one time-like dimensions. Further, the medical image data set may comprise a plurality of individual medical images.

The medical image data set comprises image data, for example, in the form of a two- or three-dimensional array of pixels or voxels. Such arrays of pixels or voxels may be representative of color, intensity, absorption or other parameters as a function of two or three-dimensional position, and may, for example, be obtained by suitable processing of measurement signals obtained by a medical imaging modality or image scanning facility.

The medical image data set may be a radiology image data set depicting a body part of a patient. Accordingly, it may contain two or three-dimensional image data of the patient's body part. The medical image may be representative of an image volume or a cross-section through the image volume. The patient's body part may be comprised in the image volume.

A medical imaging modality corresponds to a system used to generate or produce medical image data. For example, a medical imaging modality may be a computed tomography system (CT system), a magnetic resonance system (MR system), an angiography (or C-arm X-ray) system, a positron-emission tomography system (PET system) or the like. Specifically, computed tomography is a widely used imaging method and makes use of X-rays produced and detected by a spatially rotating instrument. The resulting attenuation data (also referred to as raw data) is processed by a computed analytic software producing detailed images of the internal structure of the patient's body parts. The produced sets of images are called CT-scans which may constitute multiple series of sequential images to present the internal anatomical structures in cross sections perpendicular to the axis of the human body. Magnetic Resonance Imaging (MRI), to provide another example, is an advanced medical imaging technique which makes use of the effect magnetic field impacts on movements of protons. In MRI machines, the detectors are antennas, and the signals are analyzed by a computer creating detailed images of the internal structures in any section of the human body.

Accordingly, the depicted body part of the patient in general will comprise a plurality of anatomies and/or organs. Taking a chest image as an example, the medical image may show lung tissue, the rib cage, lymph nodes and others.

A medical image data set may comprise a plurality of images or image slices. The slices respectively show a cross-sectional view of the image volume. The slices may comprise a two-dimensional array of pixels or voxels as image data. The arrangement of slices in the medical image data set may be determined by the imaging modality or by any post-processing scheme used. Further, slices may artificially be defined in the imaging volume spanned by the medical image data set. Optionally, this may happen as a function of the image data comprised in the medical image data set in order to optimally pre-process the medical image data set for the ensuing diagnostic workflow.

The medical image may be stored in a standard image format such as the Digital Imaging and Communications in Medicine (DICOM) format and in a memory or computer storage system such as a Picture Archiving and Communication System
(PACS), a Radiology Information System (RIS), and the like. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

According to some examples, the supplementary information may be non-image data of the patient. According to some examples, the supplementary information comprises structured and/or un-structured natural language text.

According to some examples, the supplementary information comprises one or more of the following elements:
- a prior medical report of the patient, and/or
- a-priori knowledge of at least one type of medical problem the patient is suspected to have, and/or
- an indication of a diagnostic task to be performed based on the medical image data set for the patient, and/or
- a medical guideline applicable for the patient, and/or
- an electronic health record of the patient.

According to some examples, the electronic health record may comprise the patient history of the patient including any pre-existing illnesses, comorbidities, risk factors, referral letters, demographic information such as age or gender, and the like.

Obtaining the supplementary information may comprise querying a healthcare information system such as a HIS (hospital information system), a LIS (laboratory information system), an EMR-system (electronic medical record system) and the like for supplementary information of the patient. Such supplementary information may be obtained in the form of one or more EMR-files (electronic medical record-files), for instance. Further, querying healthcare information systems may be based on a patient identifier such as an ID or the patient's name, electronically identifying the patient in the system.

A compartment may generally relate to an entity of the patient's organism. For instance, a compartment may relate to an organ, an organ part, an organ function, an anatomic structure, an anatomy, a functional unit of the patient's organism and so forth.

The text generation function may be configured to recognize and/or understand natural language and, in particular, individual items such as words in input containing natural language, and to transfer the items into a text output. The natural language processing algorithm may be based on a trained or machine-learned function. As an alternative, the natural language processing algorithm may be rule-based.

According to some examples, the text generation function is configured to identify data elements (also denoted as excerpts) in the supplementary information relating to the specific compartment, and to generate the text summary for the specific compartment based on the identified data elements. According to some examples, the text generation function is configured to filter the data elements for relevant information, and to generate the text summary for the specific compartment based on the relevant information.

According to some examples, the text generation function is configured to process or alter the data elements and generate the text summary based on the altered data elements. According to some examples, altering may comprise linguistic processing such as rewording, translating, shorten, and the like.

Providing the text generation function may comprise holding the function available in a suitable storge accessible by a computing unit executing the method. Further, providing the function may comprise downloading it by said computing unit.

According to some examples, the text summary may comprise structured or unstructured natural language text. According to some examples, the text summary may have a predefined format and/or text length. According to some examples, the text summary comprises an excerpt of the supplementary information According to some examples, the format may comprise one or more bullet points with information pertaining to the compartment. According to some examples, the text length may comprise a predefined number of sentences such as one to ten sentences, in particular, two, three, or four sentences. According to some examples, the text summary has a shorter text length than the text comprised in the supplementary information. According to some examples, the text summary may comprise one or more medical findings for the compartment.

According to some examples, the text summary may be displayed to the user in the user interface. According to some examples, the text summary may be displayed in a manner that it is attributable to the underlying compartment by the user. According to some examples, the text summary may be shown as an overlay over the compartment in a visualization (or representation) of the medical image data set in the user interface. According to some examples, the displaying of the text summary may be activated/initiated by the user, for instance, by hovering over the compartment with a mouse cursor in the visualization of the medical image data set.

According to some examples, providing may comprise highlighting that a text summary is available for the identified compartment in a visualization (or representation) of the medical image data set showing the identified compartment.

By generating a text summary for a specific compartment, the user is provided with a quick overview about clinically relevant information regarding the compartment. The generation of the text summary can be conceived as deriving a medical diagnosis based on the automated processing of medical information (i.e., the supplementary information). This spares the user from having to search the patient file for relevant information. In addition, since the text generation is based on what is actually shown in the medical image data set, the processing optimally complements the diagnostic task a user has to perform based on the medical image data set.

According to some examples, the step of identifying the compartment may comprise subjecting the medical image data set to an image processing step configured to identify one or more compartments in medical image data.

According to some examples, the image processing step may comprise an image segmentation step. Generally, as mentioned, any depicted body part will comprise a plurality of compartments such as, taking a chest image as an example, lung lobes, the bone compartment (e.g., comprising the rib structure or the spine), the heart, hardware such as implants, pacemakers, or catheters, and so forth. According to some examples, the step of segmenting may comprise segmenting a plurality of (different) compartments so as to provide image data respectively pertaining to the segmented compartments.

In principle, a plethora of functionalities and methods is known for image segmentation in medicine - all of which may be implemented in the segmentation steps as herein described. For instance, reference is made to US 7,822,274 B2, US 9,760,807 B2, US 11,393,229 B2 the contents of which are incorporated herein in their entirety by reference.

According to some examples, the image processing step may comprise an image recognition or classification step configured to classify image data according to its affiliation to a certain compartment. For instance, image classifiers may be extracted and compared to signatures of different compartments. For instance, reference is made to US 2023 / 0 041 553 A1 the contents of which are incorporated herein in their entirety by reference.

According to some examples the supplementary information comprises at least one medical report of the patient comprising structured and/or unstructured text.

According to some examples the supplementary information comprises a plurality of different medical reports of the patient comprising structured and/or unstructured text and the text summary summarizes the plurality of different medical reports.

In other words, according to the above examples, the text summary may be conceived as a compartment-specific text summary of one or more medical reports of the patient. Accordingly, the user does not have to read the individual reports but is automatically provided with summaries which are structured according to the contents of the medical image data set.

According to some examples, the step of identifying the at least one compartment comprises: obtaining an indication of a disease pattern for the patient, performing a lookup operation in an association linking compartments to specific disease patters so as to determine the at least one compartment. According to some examples, the step of identifying may also comprise identifying the determined compartment in the medical image data set (e.g., by applying a corresponding segmentation algorithm).

According to some examples, the indication may comprise one or more of the following: a reason for the medical image data set having been acquired, a diagnostic task to be performed based on the medical image data set, one or more suspected diagnoses, one or more proven diagnoses, and the like.

According to some examples, the indication may be obtained based on a direct input by the user. According to other examples, the indication may be automatically identified in the supplementary information and/or the medical image data set. According to some examples, this may comprise applying the text generation function to the supplementary information and/or the medical image data set which text generation function may then be additionally configured to extract indications of a disease pattern from supplementary information and/or the medical image data set.

According to some examples, the (electronic) association may comprise one or more potentially affected compartments for at least one disease pattern. In other words, the association links disease patterns to potentially affected organs. This may be based on clinical experience, e.g., outlining metastasis pathways. Accordingly, potentially relevant compartments may be automatically identified which allows for the targeted generation of text summaries.

According to some examples, the association may be based on or comprise one or more clinical ontologies such as RadLex or SNOMED. According to some examples, the association may comprise a medical guideline.

According to some examples, the text generation function is configured to filter the supplementary information and/or the data elements according to a time of creation (i.e., a time when the respective piece of supplementary information and/or the data elements were created). Specifically, according to some examples, the text generation function may be configured to filter the supplementary information and/or the data elements for elements which were created in a specific time interval. According to some examples, the time interval may extend from a first point in time to a second point in time. According to some examples, the second point in time may be now and the first point in time may be a predetermined point in time in the past.

Accordingly, the acquisition time of a certain piece of information may be used as a parameter for retrieving relevant information from the supplementary information. With that, outdated information may automatically be discarded.

According to an aspect, the text generation function, comprises a transformer network and/or a large language model.

A transformer network is a neural network architecture generally comprising an encoder, a decoder or both an encoder and decoder. In some instances, the encoders and/or decoders are composed of several corresponding encoding layers and decoding layers, respectively. Within each encoding and decoding layer is an attention mechanism. The attention mechanism, sometimes called self-attention, relates data items (such as words) within a series of data items to other data items within the series. The self-attention mechanism for instance allows the model to examine a group of words within a sentence and determine the relative importance other groups of words within that sentence have to the word being examined.

The encoder, in particular, may be configured to transform the input (text) into a numerical representation. The numerical representation may comprise a vector per input token (e.g., per word). The encoder may be configured to implement an attention mechanism so that each vector of a token is affected by the other tokens in the input. In particular, the encoder may be configured such that the representations resolve the desired output of the transformer network.

The decoder, in particular, may be configured to transform an input into a sequence of output tokens. In particular, the de-coder may be configured to implement a masked self-attention mechanism so that each vector of a token is affected only by the other tokens to one side of a sequence. Further, the de-coder may be auto-regressive meaning in that intermediate results (such as a previously predicted sequence of tokens) are fed back.

According to some examples, the output of the encoder is input into the decoder.

Further, the transformer network may comprise a classification module or unit configured to map the output of the encoder or decoder to a set of learned outputs such as the text summary.

Training of a transformer model according to some examples may happen in two stages, a pretraining and a fine-tuning stage. In the pretraining stage, a transformer model may be trained on a large corpus of data to learn the underlying semantics of the problem. Such pre-trained transformer models are available for different languages. For certain applications described herein, the fine-tuning may comprise further training the transformer network with medical texts with expert annotated meanings and/or medical ontologies such as RADLEX and/or SNOMED. With the latter, in particular, the transformer model according to some examples may learn typical relations and synonyms of medical expressions.

For a review on transformer networks, reference is made to Vaswani et al., "Attention Is All You Need", in arXiv: 1706.03762, June 12, 2017, the contents of which are herein included by reference in their entirety.

An advantage of transformer networks is that, due to the attention mechanism, transformer networks can efficiently deal with long-range dependencies in input data. Further, encoders used in transformer networks are capable of processing data in parallel which saves computing resources in inference. Moreover, decoders of transformer networks, due the auto-regression, are able to iteratively generate a sequence of output tokens with great confidence.

According to an aspect, the step of identifying comprises identifying a plurality of different compartments in the medical image data set, and the step of applying comprises applying the text generation function to the supplementary information so as to generate a respective text summary for each of the plurality of different compartments.

According to some examples, the method further comprises generating (rendering) a representation of the medical image data set for displaying to the user via the user interface, and displaying the representation to the user via the user interface, wherein the respective text summaries are generated prior to displaying the representation.

The representation may be a two-dimensional representation image rendered from the medical image data set. The representation may comprise a plurality of image pixels. In particular, the representation may be a two-dimensional rendering of the medical image. Two-dimensional renderings may, in general, rely on known rendering procedures, such as ray-casting, ray-tracing, texture-rendering or the like. According to some examples, the rendering may be such that already identified compartments are displayed in conjunction with the image data of the medical image.

By creating a text summary for a plurality of compartments, relevant information can be held available for the user which she or he can access/display on a request basis. In particular, by already generating the text summaries prior to displaying a rendering of the medical image study, the text summaries are ready already available before the user inspects the case. With that, the method seamlessly integrates into the workflow.

According to an aspect, the method further comprises receiving a request for a holistic text summary for the medical image data set, generating a holistic text summary by applying the text generation function to the respective text summaries, wherein the holistic text summary comprises a synopsis of the respective text summaries.

In other words, a "summary of the summaries" is generated. This provides the user with an overview of the entire case which she or he can trace back to individual text summaries for individual compartments. What is more, by basing the holistic summary on the text summaries of the individual compartments, the holistic summary is intrinsically structured which makes it better accessible for the user.

According to some examples, the step of generating the holistic text summary comprises determining a weighting for each of the text summaries, wherein the holistic summary reflects the weightings. According to some examples, the weighting comprises a weighting according to a relevance level (optionally according to a relevance level as described below), wherein the relevance level may indicate a clinical relevance of the respective text summary.

According to an aspect, the method further comprises classifying the plurality of different compartments based on the text summaries (the step of generating the respective text summaries) according to at least two relevance levels (or criticality levels) so as to provide a classification result for each of the plurality of compartments, optionally wherein one of the relevance levels indicates the absence of any medically relevant findings in the corresponding compartment, and providing the classification results to the user via the user interface.

In other words, a classification based on the natural language text of the text summaries is proposed (and not, e.g., based on image data of the medical image data set). According to some examples, the step of classifying may be performed by the appropriately configured text generation function. According to other examples, the step of classifying may be performed by a separate classification function configured to classify text into different relevance levels.

The classifying step may be based on recognizing pre-defined trigger words in the respective text summaries, which trigger words may indicate a certain relevance or criticality of findings comprised in the respective text summaries.

According to some examples, in the step of classifying, compartments may be classified according to two relevance levels. According to some examples the two relevance levels may comprise "normal" and "not-normal".

According to other examples, the compartments may be classified according to more than two relevance levels, and, in particular, according to a continuum of relevance levels. For instance, a number, e.g., between zero and one, may be assigned to each text summary in the step of classifying, wherein the number indicates the respective relevance or criticality level.

According to some examples, "normal" may mean that the compartment is medically inconspicuous based on the corresponding text summary. According to some examples, "normal" may mean that the text summary does not comprise (or indicate) any medical findings (or medical abnormalities).

A medical finding may indicate a certain condition or pathology of the patient which is relevant for the diagnosis of the patient, and which requires the attention of the user. A medical finding may be an anatomical structure that differentiates the patient from other patients. A medical finding may be a medical abnormality. A medical finding may be located within different organs of the patient (e.g., within the lung of a patient, or within the liver of a patient) or in between the organs of the patient. In particular, a medical finding may be a foreign body. In particular, a medical finding may be a neoplasm (also denoted as a "tumor"), in particular, a benign neoplasm, an in situ neoplasm, a malignant neoplasm and/or a neoplasm of uncertain/unknown behavior. In particular, a medical finding can be a nodule, in particular, a lung nodule. In particular, a medical finding may be a lesion, in particular, a lung lesion.

According to some examples, those compartments are classified as "not-normal" where medical findings or abnormalities are actively mentioned in the text summaries.

According to some examples, "normal" may mean that the text summary does not comprise (or indicate) any actionable medical abnormalities. This may mean no findings at all or only non-actionable findings. Non-actionable findings or abnormalities are findings which do not require any immediate action from the user. Examples for non-actionable abnormalities could be, e.g., anatomical variants, degenerative changes, e.g., bone changes in elderly patients, healed fractions, or tiny lung nodules which do not require follow-up. Other examples for such non-actionable findings which could still classify the medical image dataset as normal are "hardware" which has been imaged such as surgical clips, (neck) laces, implants, ECG leads, etc. Such findings may be assigned lower relevance or criticality levels.

According to some examples, the classification result provided in the classifying step indicates whether or not the respective text summary, as a whole, indicates that the underlying compartment is normal. According to some examples, some compartments may be normal while other compartments are not-normal.

According to some examples, the relevance levels may be provided as an overlay over a representation of the medical image data set. In particular, in the overlay, the relevance levels may be encoded in markings or in a color coding. According to some examples, providing the relevance levels may comprise to selectively show text summaries of one or more relevance levels in the user interface and hide text summaries of other relevance levels. For instance, only text summaries which were classified as not-normal may be shown.

By providing a classification result based on the text summaries, a further level of abstraction may be easily and reproducibly provided. In other words, a higher-level medical diagnosis is provided based on the further processing of the text summaries. Accordingly, the user is provided with further assistance were to focus her or his attention for providing a medical diagnosis.

According to some examples, the method further comprises generating a ranking of the text summaries, optionally based on the relevance levels, and providing the ranking.

According to some examples, the ranking may be according to a clinical relevance (e.g., the relevance level) or a criticality. According to some examples, the ranking may be provided in the form of an ordered list to the user (from which the user may select). With the ranking, the user is provided with an indication which one of the text summaries is the most pertinent one.

According to some examples, the ranking may be generated based on pre-determined rules or criticality identifiers such as the number of mentions of a certain finding for a compartment, changes (deteriorations) in the findings for a compartment over time, relevance/criticality of the compartment, relevance/criticality of the finding and the like.

According to some examples, the step of providing comprises obtaining a compartment of interest of the user, selecting the text summary pertaining to the compartment of interest from the respective text summaries, and providing the selected text summary to the user via the user interface.

The compartment of interest may be the compartment the user is currently focusing her or his attention at. Further, the compartment of interest may be a compartment, the user should consider. The compartment of interest may be obtained by monitoring the user. Further, the compartment of interest may be obtained based on the supplementary information. For instance, if the supplementary information indicates that the user is to examine a certain compartment (for instance, in the form of a diagnostic task), this compartment may be identified as the compartment of interest. According to some examples, the step of providing the selected text summary may again comprise displaying the selected text summary in the user interface.

By identifying a compartment of interest and showing the corresponding text summary, the user may be automatically provided with the relevant information. In other words, not all text summaries are provided but specifically those which are of interest for the user. With that, the user can be further relieved when screening a complex case.

According to some examples, the method further comprises obtaining at least one imaging parameter of the medical image data set, the imaging parameter relating to settings used during acquisition and/or pre- or post-processing of the medical image data set, wherein the step of obtaining the compartment of interest is further based on the imaging parameter.

According to some examples, the imaging parameter comprises one or more of the following parameters:
- a patient position during the image acquisition process,
- an image acquisition protocol used for acquiring raw data based on which the medical image data set is generated, and/or
- an image reconstruction protocol used for generating the medical image data set based on raw data.

The acquisition protocol may relate to the kind of medical imaging modality used for acquiring the medical image data or the underlying raw data. For instance, the acquisition protocol may specify if an MRI system or a CT system has been used. Further, the acquisition protocol may relate to settings used for the medical imaging modality during the acquisition. Taking an MRI system as an example, this may comprise the MR pulse sequence used.

The reconstruction protocol may relate to the reconstruction algorithm and the corresponding settings used for processing the acquired raw data so as to provide the medical image data set. Taking the CT imaging process as an example, the reconstruction protocol may specify the kernel (or convolution algorithm) used. The kernel refers to the process used to modify the frequency contents of projection data prior to back projection during image reconstruction in a CT scanner. This process corrects the image by reducing blurring. The kernel affects the appearance of image structures by sharpening the image. Different kernels have been developed for specific anatomical applications including soft tissue (standard kernel) and bone (bone kernel).

Taking imaging parameters into account upon obtaining the compartment of interest is beneficial since the imaging parameters provide additional insights into the kind of organs, anatomies, and medical findings, a medical image data set can indicate. Thus, the prediction of the compartment of interest and therewith the relevant text summary may be improved.

According to some examples, the step of obtaining the compartment of interest comprises receiving a user input into the user interface, and determining the compartment of interest based on the user input.

The user input may be any input directed to a compartment of interest. The user input may comprise a voice command or any other, in particular, manual input into a user interface, in particular, a graphical user interface. For instance, the user may use input devices like a computer-mouse, a trackball device, a smart pen, a touch pad, a touch sensitive display, etc. Further, the user input may be captured by eye tracking or by tracking gestures.

According to some implementations the user input may comprise a plurality of individual user interactions with a user interface (such as user inputs with regard to the representation, displaying settings, general settings, measurements, etc.).

By predicting the compartment of interest based on the user input, the user is automatically provided with appropriate text summaries. By consequence, the user is automatically provided with an actionable result he can immediately use in the downstream reporting workflow. Accordingly, reviewing medical images and compiling structured medical reports on that basis can be rendered considerably more efficient.

According to some examples, receiving the user input may comprise recording an audio and/or video signal of an environment of the user (e.g., with an appropriate recording device) and identifying a user input in the signal. According to some examples, the signal comprises a recording of a conversation of a plurality of users (e.g., in a tumor board situation) and the step of identifying comprises identifying one or more user inputs in the conversation. In other words, the method may "listen" to the user or a group of users and automatically select appropriate text summaries according to a current subject of a conversation or the like. Therewith the method interactively supports the user even in situations where the user is not actively making inputs into the user interface.

According to some examples, the method further comprises generating a representation of the medical image data set for displaying to the user via the user interface, displaying the representation to the user via the user interface, wherein, in the step of receiving the user input, the user input is directed to the compartment of interest visible in the representation.

According to some examples, the user input comprises an interaction with the representation. The user input may, in particular, comprise designating a compartment of interest directly in the representation, e.g., by clicking, drawing contours, or invoking a measurement tool in a specific location in the representation. The user input may be such that it fully lines out the compartment of interest or such that it indicates only parts or even only a point in the representation which is then automatically related to a compartment of interest.

By providing the representation and identifying the user interaction in the form of an interaction with the representation, a continuous human machine interaction is enabled which seamlessly integrates in the reporting workflow.

According to some examples, the method further comprises determining an anatomical location the user input is directed to based on the user input and the representation and/or the medical image data set. Further, the step of determining the compartment of interest is based on the anatomical location.

According to some examples, the anatomical location comprises one or more of the following elements:
- an indication of an organ or organ part the user input is directed to,
- a slice within the medical image data set, the representation corresponds to, and/or
- a relation of the user input to one or more anatomical landmarks.

With the anatomical location, information may be retrieved indicating the anatomic context of the user input and, therewith, the compartment of interest. With that, the compartment of interest can be identified yielding better results in the prediction stage as well as in the downstream reporting process.

According to an aspect, the method further comprises obtaining one or more displaying settings applied by the user for the representation upon displaying the representation, and the step of obtaining the compartment of interest is further based on the displaying settings.

According to an aspect, the displaying settings comprise one or more of the following settings:
- an organ segmentation applied to the representation,
- an intensity windowing applied to the representation,
- contrast and/or brightness adjustments applied to the representation
- a look-up table applied to the representation,
- an auto-view setting applied to the representation,
- a viewing plane applied to the representation, and/or
- a zoom level or panning applied to the representation.

Taking CT image data as an example, the representation may be intensity filtered. Each voxel of a CT image data set usually has an intensity value that represents the attenuation of X-rays at the corresponding position in the volume as determined from the CT measurements (commonly measured in Hounsfield or HU units). Due to this relation, a kind of segmentation can be performed based solely on a thresholding of the voxel intensity values. For example, all voxels having intensity values in a particular range may be considered to represent bone. Other windows may relate to carve out soft tissue or lung parts. In the art, these windows are commonly referred to as HU-windows. For instance, if the bone window is applied, it can be concluded that the compartment of interest may relate to the skeletal structure of the patient.

An auto-viewing setting may, in particular, relate to a setting for a particular diagnostic task or a particular organ. The auto-viewing setting may be provided by the user interface for selection by the user. Since the auto-viewing settings are specific to a certain diagnostic task or anatomy, their knowledge may provide insights into the likely compartment of interest.

In particular, the viewing plane may be selected from an axial or sagittal or coronal viewing plane. Generally speaking, it relates to the viewing plane under which the medical image data set (or the underlying image volume) is being looked at. In the diagnostic workflow, the viewing direction already is an indication for the compartment a user is interested in.

In general, taking display or viewing settings into account upon obtaining the compartment of interest is beneficial, as such display settings provide valuable insights into the kind of organs, anatomies and medical findings, a user is interested in.

According to some implementations, the step of determining an anatomical location may further be based on the display settings.

According to an aspect, the user input is directed to generate a measurement of an image feature depicted in the representation, and the step of obtaining the compartment of interest is further based on the measurement.

For instance, the measurement may be a volume, surface, angle, or distance in the representation. Further, the measurement may involve defining a region of interest in the representation. In addition, the measurement may be based on image data related to the user input. For instance, an intensity profile (e.g., measured in Hounsfield units) of such image data may indicate if a certain medical finding relates to a cyst or a malignancy.

As the measurement may indicate the compartment, taking the measurement into account upon predicting the compartment of interest can improve the outcome.

According to an aspect, generating the measurement involves selecting an image measurement tool from a plurality of available image measurement tools by the user, and the step of predicting the compartment of interest is further based on the measurement tool selected by the user.

In general, there will be various kinds of measurement tools available for the user. For instance, such tools may be geometry measurement tools, volume measurement tools, image processing tools, and/or computer aided detection tools. Accordingly, the kind of tool invoked by the user also indicates the compartment of interest. Thus, using this information in the prediction step may further improve the outcome.

According to an aspect, the text summary has a predetermined text length, and the step of providing comprises receiving a request from the user to provide additional details, and expanding the text summary into a text of a text length longer than the predetermined text length based on the supplementary information using the text generation function.

In other words, upon request, additional information may be pulled from the supplementary information and integrated into an extended version of the text summary. This additional information may be information which was previously disregarded for the more condensed initial version of the text summary. For instance, if the text summary contains a certain number of bullet points and/or sentences, the expanded text summary may comprise additional bullet points or sentences. Accordingly, the user may be provided with additional information only where she or he explicitly requests it. With that, different levels of detail may be provided in a continuous human-machine interaction without overburdening the user with an information overflow.

According to an aspect, the method further comprises receiving a natural language query from the user regarding the text summary, inputting the natural language query into the text generation function so as to generate a natural language answer to the natural language query by applying the text generation function to the supplementary information and/or general information, outputting the natural language answer to the user (e.g., via the user interface).

According to some examples, the general (medical) information may be medical information not specific for the patient. The general information may be valid for a plurality of patients and/or a cohort of patients. According to some examples, the general information may comprise one or more medical guidelines and/or one or more electronic textbooks.

The user queries may be free text queries the user may input into a user interface, e.g., by typing the query into an appropriate input field in the user interface or by voice command. The queries may ask for additional information from the EMR of the patient. In addition, the queries may ask for more general information which can be answered by taking the general information into account. For example, the user queries may be of the following kind: "show me additional information regarding finding XY", "what was the size of finding XY in the prior exam", "what kind of therapies have already been prescribed", "what kind of therapies come into question", "who is the referring physician" and the like.

By enabling free text queries from the user, the user can easily request additional information without requiring the user to directly interact with data. This simplifies the workflow and reduces the workload for the user.

According to an aspect, the step of providing the text summary comprises obtaining, for at least one item in the text summary, an electronic link to the source of the item in the supplementary information, wherein the link is configured to enable retrieving the source in the user interface, and including the link in the text summary.

The source may be a data element comprised in the supplementary information such as a document like a medical report, an image data set, a non-image data set such as lab values, and the like.

Providing the link enables to quickly retrieve the source document which forms the basis for a certain item in the text summary. In turn, this enables the user to verify a given element in the text summary and gather additional information by inspecting the source. Furthermore, this provides an explanation as to why a certain item was included in the text summary.

According to some examples, the method further comprises displaying a representation of the source in the user interface. According to some examples, displaying the representation of the source comprises: receiving a user input directed to display the source, and displaying the representation of the source as a response to the user input. The representation of the source may be a rendering of the source such as a rendering of a medical image data set or a document.

According to an aspect, the text generation function is further configured to detect a change over time of one or more items of the text summary, and the step of providing the text summary comprises indicating the change for the user.

By detecting the change, a deterioration or improvement of a certain condition of a patient can be identified.

According to some examples, the change may be determined based on a time stamp of individual elements in the supplementary information. For instance, a reference text summary may be generated based on the data elements generated before a certain cutoff date, i.e., the text generation function may be applied to only such data elements. Then, the text generation function may be applied to all data elements so as to generate the text summary. Recent changes may then be determined by comparing the text summary with the reference text summary.

According to an aspect, a system for providing a text summary is provided. The system comprises an interface unit and a computing unit. The interface unit is configured to receive a medical image data set of a patient, to access supplementary information associated to the patient in a medical information system, and to provide the text summary to a user. The computing unit is configured to identify at least one compartment in the medical image data set, to provide a text generation function configured to provide, for a specific compartment, a natural language text summary summarizing medical information pertaining to the specific compartment, and to apply the text generation function to the supplementary information so as to generate a text summary for the at least one compartment.

The computing unit may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories and combinations thereof. The one or more memories may store instructions for carrying out the method steps according to the invention. The hardware can be configurable by the software and/or be operable by the software. Generally, all units, sub-units, or modules may at least temporarily be in data exchange with each other, e.g., via a network connection or respective interfaces. Consequently, individual units may be located apart from each other.

The interface unit may comprise an interface for data exchange with a local server or a central web server via internet connection for receiving the medial image data sets. The interface unit may be further adapted to interface with one or more users of the system, e.g., by displaying the result of the processing by the computing unit to the user (e.g., in a graphical user interface) or by allowing the user to adjust parameters for data processing or visualization. In other words, the interface unit may comprise a user interface.

According to other aspects, the invention further relates to an integrated data management system comprising the above system and the medical information system. Further, such integrated data management system may comprise an image archiving system configured to acquire, store and/or forward medical images. Thereby, the interface unit may be configured to receive the medical image data set form the image archiving system. According to some examples, the image archiving system may be realized as a cloud storage or as a local or spread storage, e.g., as a PACS (Picture Archiving and Communication System). According to other aspects, the systems are adapted to implement the inventive method in their various aspects for providing a candidate medical finding. The advantages described in connection with the method aspects may also be realized by the correspondingly configured systems' components.

According to another aspect, the present invention is directed to a computer program product comprising program elements which induce a computing unit of a system configured to provide a text summary for a medical image data set to perform the steps according to one or more of the above method aspects, when the program elements are loaded into a memory of the computing unit.

According to another aspect, the present invention is directed to a computer-readable medium on which program elements are stored that are readable and executable by a computing unit of a system for providing a text summary for a medical image data set according to one or more method aspects, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:
- FIG. 1: schematically depicts a system for providing a text summary according to an embodiment,
- FIG. 2: schematically depicts a method for providing a text summary according to an embodiment,
- FIG. 3: schematically depicts data flows in a method for providing a text summary according to an embodiment,
- FIG. 4: schematically depicts optional method steps in a method for providing a text summary according to an embodiment,
- FIG. 5: schematically depicts data flows in a method for providing a summary according to an embodiment, and
- FIG. 6: schematically depicts a text generation function according to an embodiment.

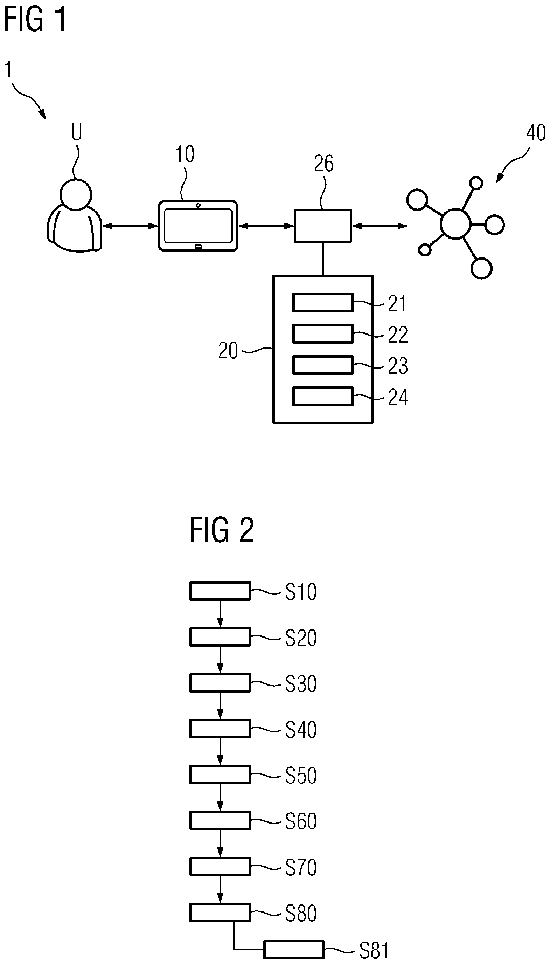

Figure 1 depicts a system 1 for providing a text summary TS in connection with a medical image data set MIDS for a user U. In this regard, system 1 is adapted to perform the methods according to one or more embodiments, e.g., as further described with reference to Figures 2 to 5.

The user U of system 1 (in the sense of an operator controlling the system 1), according to some examples, may generally relate to a healthcare professional such as a physician, clinician, technician, radiologist and so forth.

System 1 comprises a user interface 10 (as part of the interface unit) and a processing system 20 (as part of the computing unit). Further, system 1 may comprise or be connected to a medical information system 40. The medical information system 40 may generally be configured for acquiring and/or storing and/or forwarding medical image data sets MIDS and supplementary information SI. For instance, medical information system 40 may comprise one or more archive/review station (not shown) for medical image data sets MIDS. The archive/review stations may be embodied by one or more databases. In particular, the archive/review stations may be realized in the form of one or more cloud storage modules. Alternatively, the archive/review stations may be realized as a local or spread storage, e.g., as a PACS (Picture Archiving and Communication System). According to some examples, medical information system 40 may also comprise one or more medical imaging modalities (not shown), such as a computed tomography system, a magnetic resonance system, an angiography (or C-arm X-ray) system, a positron-emission tomography system, a mammography system, an X-ray system, or the like.

Medical image data sets MIDS may be three-dimensional image data sets acquired, for instance, using an X-ray system, a computed tomography system or a magnetic resonance imaging system or other systems. The image information may be encoded in a three-dimensional array of m times n times p voxels. Medical image data sets MIDS may include a plurality of image slices which are stacked in a stacking direction to span the image volume covered by the medical image data sets MIDS.

Further, medical image data sets MIDS may comprise two-dimensional medical image data with the image information being encoded in an array of m times n pixels. According to some examples, these two-dimensional medical images may have been extracted from three-dimensional medical image data sets MIDS.

An ensemble of voxels or pixels may be designated as image data of the respective medical data set MIDS in the following. Generally, medical data sets MIDS may show a body part or an anatomical region or an anatomic object of a patient which may comprise various anatomies and organs.

Medical image data sets MIDS may be formatted according to the DICOM format. DICOM (=Digital Imaging and Communications in Medicine) is an open standard for the communication and management of medical imaging information and related data in healthcare informatics. DICOM may be used for storing and transmitting medical images and associated information enabling the integration of medical imaging devices such as scanners, servers, workstations, printers, network hardware, and picture archiving and communication systems (PACS). It is widely adopted by clinical syndicates, hospitals, as well as for smaller applications like doctors' offices or practices. A DICOM data object consists of a number of attributes, including items such as the patient's name, ID, etc., and also special attributes containing the image pixel data and metadata extracted from the image data.

Supplementary information SI (or associate data) may be any data providing additional information relating to the patient and/or the medical image data set MIDS. The supplementary information SI may comprise image data such as other medical image studies of the patient which were, for instance, acquired at an earlier point in time than the medical image data set MIDS under consideration. Further the supplementary information SI may comprise non-image data or data with mixed-type contents comprising medical images and non-image contents such as text. Non-image data may relate to non-image examination results such as lab data, vital signs records (comprising, e.g., ECG data, blood pressure values, ventilation parameters, oxygen saturation levels) and so forth. Moreover, the supplementary information SI may comprise structured and unstructured medical text reports MTR relating to prior examinations or the current examination of the patient. Further, non-image data may comprise personal information of the patient such as gender, age, weight, insurance details, and so forth.

The supplementary information SI may be available in the form of one or more electronic medical records (EMR) of the patient. The supplementary information SI may be stored in the healthcare information system 40. For instance, the supplementary information SI may be stored in dedicated databases of the healthcare information system 40 such as laboratory information system (LIS) or an electronic health/medical record database.

User interface 10 may comprise a display unit and an input unit. User interface 10 may be embodied by a mobile device such as a smartphone or tablet computer. Further, user interface 10 may be embodied as a workstation in the form of a desktop PC or laptop. The input unit may be integrated in the display unit, e.g., in the form of a touch screen. As an alternative or in addition to that, the input unit may comprise a keyboard, a mouse or a digital pen and any combination thereof. The display unit may be configured for displaying a representation R of the medical image data set MIDS, for one displaying or more text summaries TS, and for receiving any user input INP, e.g., for designating a compartment of interest COI in the medical image data set MIDS.

User interface 10 may further comprise an interface computing unit configured to execute at least one software component for serving the display unit and the input unit in order to provide a graphical user interface for allowing the user U to select a target patient's case to be reviewed and making various inputs. In addition, the interface computing unit may be configured to communicate with medical information system 40 or processing system 20 for receiving the medical data sets MIDS, any supplementary information SI, and text summaries TS. The user U may activate the software component via user interface 10 and may acquire the software component, e.g., by downloading it from an internet application store. According to an example, the software component may also be a client-server computer program in the form of a web application running in a web browser. The interface computing unit may be a general processor, central processing unit, control processor, graphics processing unit, digital signal processor, three-dimensional rendering processor, image processor, application specific integrated circuit, field programmable gate array, digital circuit, analog circuit, combinations thereof, or other now known devices for processing image data. User interface 10 may also be embodied as a client.

Processing system 20 may comprise sub-units 21-24 configured to process the medical image data sets MIDS and supplementary information SI in order to provide text summaries TS for specific compartments CP shown in the medical image data sets MIDS.

Processing system 20 may be a processor. The processor may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. The processor may be single device or multiple devices operating in serial, parallel, or separately. The processor may be a main processor of a computer, such as a laptop or desktop computer, or may be a processor for handling some tasks in a larger system, such as in the medical information system or the server. The processor is configured by instructions, design, hardware, and/or software to perform the steps discussed herein. The processing system 20 may be comprised in the user interface 10. Alternatively, processing system 20 may comprise a real or virtual group of computers like a so called `cluster' or 'cloud'. Such server system may be a central server, e.g., a cloud server, or a local server, e.g., located on a hospital or radiology site. Further, processing system 20 may comprise a memory such as a RAM for temporally loading the medical image data sets MIDS. According to some examples, such memory may as well be comprised in user interface 10.

Sub-unit 21 is a data retrieval module or unit. It is configured to access and search the medical information system 40 for medical image data sets MIDS and supplementary information SI. Specifically, sub-unit 21 may be configured to formulate search queries and parse them to the medical information system 40. According to some examples, the search queries may be based on an electronic patient identifier of the patient in the medical information system 40.

Sub-unit 22 may be conceived as a segmentation module or unit. Sub-unit 22 may be configured to identify a plurality of compartments CP in the medical image data set MIDS. To this end, segmentation unit 22 may be configured to run one or more appropriate detection or segmentation algorithms.

Sub-unit 23 may be configured as a user interaction module or unit. Sub-unit 23 may be configured to provide a representation R for displaying to the user U via the user interface 10. The representation R can be in the form of a rendering of the medical image data set MIDS. Further, sub-unit 23 may be configured to identify a compartment of interest COI in the representation R (and, therewith, in the medical image data set MIDS) based on a corresponding input INP by the user U. For instance, sub-unit 23 may be configured to provide a corresponding tool, the user U may activate via the user interface 10. After the tool has been activated, user inputs INP such as speech, gesture, eye movement, handling of input devices such as computer mouses, etc. may be evaluated to derive the compartment of interest COI the user U is currently reviewing. According to an example, such a user input INP may designate a point or group of points in the representation R which is then further processed to define the corresponding compartment of interest COI. According to other examples, the tool may be a measurement tool with which the user U may obtain a measurement from the representation R. The measurement may relate to a volume, surface, angle, distance, etc.

Further, sub-unit 23 may be configured to provide text summaries TS corresponding to the identified compartments CP - and in particular, corresponding to the compartment(s) of interest COI - to the user U, e.g., in the form of a pup-up window over the representation R at the location of the respective compartment CP.

Further, sub-unit 23 may be configured to also receive and process other kinds of user inputs to control the method and allow for a continued and guided human-machine interaction. Such user inputs may relate to queries for additional information based on the text summaries TS or other queries.

Sub-unit 24 may be conceived as a text generation module or unit. It is configured to filter the supplementary information SI for information relevant for a certain compartment CP and generate a text summary TS for that certain compartment on that basis. In other words, sub-unit 24 may be configured to extract information from the supplementary information SI and transfer this information into text suited for a concise text summary TS in a compartment-specific manner. To this end, submodule 24 may run a correspondingly configured text generation function LLM.

The designation of the distinct sub-units 21-24 is to be construed by way of example and not as a limitation. Accordingly, sub-units 21-24 may be integrated to form one single unit (e.g., in the form of "the computing unit") or can be embodied by computer code segments configured to execute the corresponding method steps running on a processor or the like of processing system 20. The same holds true with respect to the interface computing unit. Each sub-unit 21-24 and the interface computing unit may be individually connected to other sub-units and/or other components of the system 1 where data exchange is needed to perform the method steps.

Processing system 20 and the interface computing unit(s) together may constitute the computing unit of the system 1. Of note, the layout of this computing unit, i.e., the physical distribution of the interface computing unit and sub-units 21-24 is, in principle, arbitrary. Specifically, processing system 20 may also be integrated in user interface 10. As already mentioned, processing system 20 may alternatively be embodied as a server system, e.g., a cloud server, or a local server, e.g., located on a hospital or radiology site. According to such implementation, user interface 10 could be designated as a "frontend" or "client" facing the user U, while processing system 20 could then be conceived as a "backend" or server. Communication between user interface 10 and processing system 20 may be carried out using the https-protocol, for instance. The computational power of the system may be distributed between the server and the client (i.e., user interface 10). In a "thin client" system, the majority of the computational capabilities exists at the server. In a "thick client" system, more of the computational capabilities, and possibly data, exist on the client.

Individual components of system 1 may be at least temporarily connected to each other for data transfer and/or exchange. User interface 10 communicates with processing system 20 via (data) interface 26 to exchange, e.g., medical image data sets MIDS, representations R, text summaries TS, or any user input INP made. For example, processing system 20 may be activated on a request-base, wherein the request is sent by user interface 10. Further, processing system 20 may communicate with medical information system 40 in order to retrieve a target patient's case. As an alternative or in addition to that, user interface 10 may communicate with medical information system 40 directly. Medical information system 40 may likewise be activated on a request-base, wherein the request is sent by processing system 20 and/or user interface 10. Data interface 26 for data exchange may be realized as hardware- or software-interface, e.g., a PCI-bus, USB or fire-wire. Data transfer may be realized using a network connection. The network may be realized as local area network (LAN), e.g., an intranet or a wide area network (WAN). Network connection is preferably wireless, e.g., as wireless LAN (WLAN or Wi-Fi). Further, the network may comprise a combination of different network examples. Interface 26 for data exchange together with the components for interfacing with the user U be regarded as constituting an interface unit of system 1.

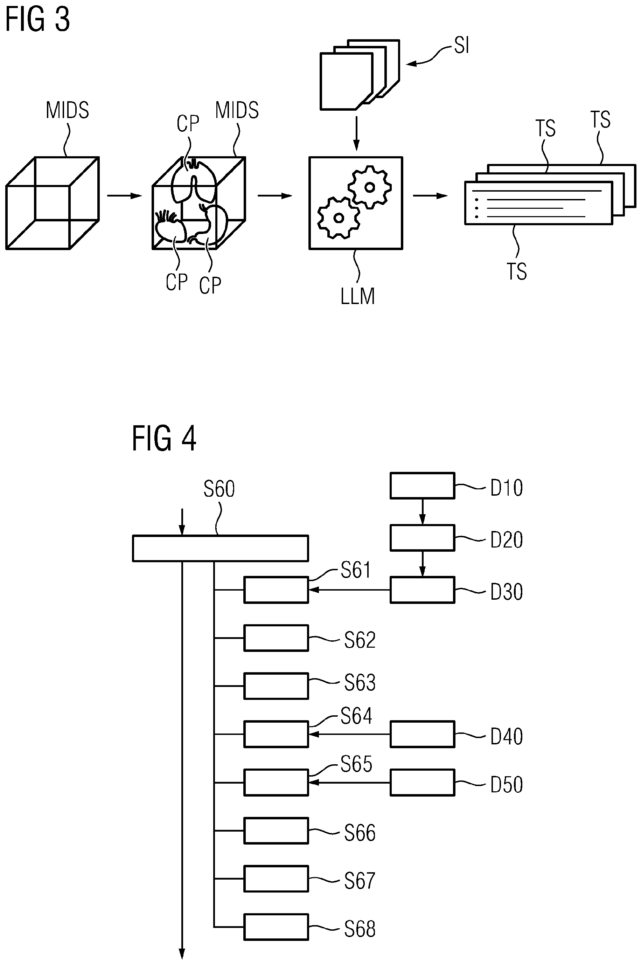

Figure 2 depicts a method for providing a text summary TS according to an embodiment. Corresponding data streams are illustrated in Figure 3. The method comprises several steps. The order of the steps does not necessarily correspond to the numbering of the steps but may also vary between different embodiments of the present invention. Further, individual steps or a sequence of steps may be repeated.

In a first step S10, the medical image data set MIDS is received. This may involve selecting the medical image data set MIDS from a plurality of cases, e.g., stored in the medical information system 40. The selection may be performed manually by the user U, e.g., by selecting appropriate image data in a graphical user interface running in the user interface 10. Alternatively, the medical image data set MIDS may be provided to the computing unit by the user U by way of uploading the medical image data set MIDS to the computing unit.

At step S20, one or more compartments CP are identified in the medical image data set MIDS. The compartments CP identified may be organs, anatomical structures or functional units of the patient's body which are at least partially shown in the medical image data set MIDS. According to some examples, all or at least all relevant compartments CP shown in the medical image data set MIDS may be identified at step S20.

In order to identify the compartments CP, step S20 may involve applying one or more appropriately configured detection algorithm to the medical image data set MIDS. The detection algorithms may comprise medical image segmentation algorithms configured to identify and segment particular compartments in medical image data. Further, the detection algorithms may comprise image classification algorithms configured to identify certain compartments based on image features extracted from the medical image data set MIDS. Thereby, step S20 may comprise applying a plurality of different detection algorithms to the medical image data set MIDS. Further, step S20 may comprise selecting one or more detection algorithms from a plurality of available detection algorithms and applying the selected detection algorithms to the medical image data set MIDS. According to some examples, the selection may be based on a body region shown in the medical image data set MIDS or an indication of which compartments CP could be of particular interest. The latter may be based on user inputs in the system 1 or on a notion of a reason for the exam (i.e., the medical image data set MIDS).

At step S30, supplementary information SI is accessed for the patient. The supplementary information SI may be the entirety of information available for the patient in the medical information system 40. The supplementary information SI may comprise non-image data and, in particular, one or more structured or un-structured (text-)documents which were generated at different points in time in the past.

The supplementary information SI may be obtained by querying the medical information system 40, e.g., based on an electronic patient identifier of the patient in the medical information system 40.

At step S40, the text generation function LLM is provided. For instance, the text generation function LLM may be provided by holding it available in a memory of the processing system 20, for instance, as executable computer code.

The text generation function LLM is configured to parse patient information (such as the supplementary information SI) for information related to a particular compartment CP. Further, the text generation function LLM is configured to condensate the information thus retrieved in a text summary text TS which may have a pre-defined maximal text length, e.g., a certain number of bullet points.

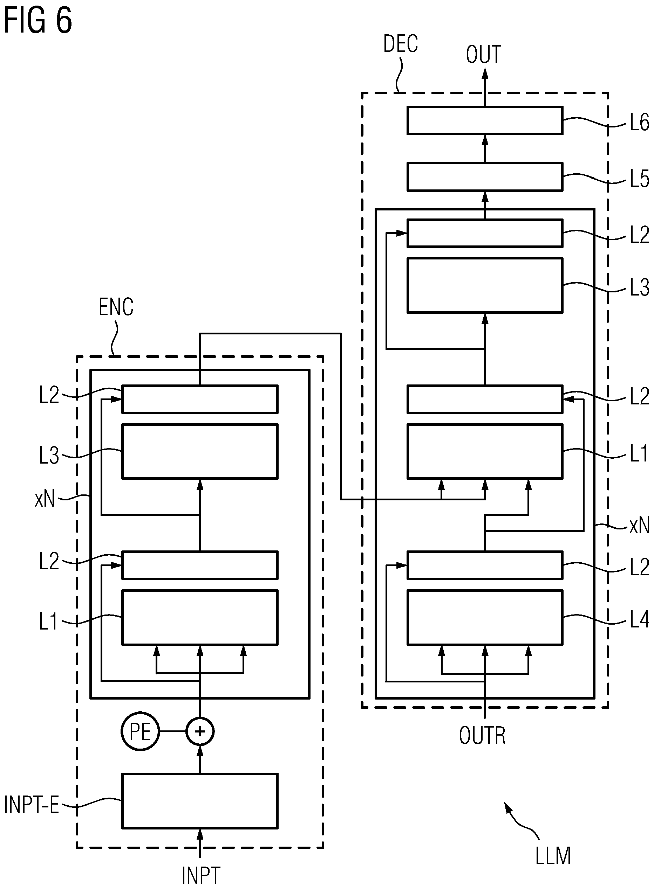

In general, the text generation function LLM may be a trained function which was configured for the task to generate text summaries TS in a dedicated training process. According to some examples, the text generation function LLM may have been trained using compartment-specific text summaries TS generated by experts. Specifically, in the training process, text summaries TS generated by the text generation function LLM may be compared to the expert text summaries TS. Based on the comparison, parameters of the text generation function LLM may be adapted so that the text generation function LLM better reflects the desired behavior as specified by the expert text summaries TS. That way, the text generation function LLM may learn which data items in the supplementary information SI are relevant for which compartment CP and which data items are more important than others and should be prioritized for the text summary TS. A more concrete example of the text generation function LLM is provided in connection with Figure 6.

According to some examples, a prioritization of information for a text summary TS may be based on the point in time the data was generated (new data over old data) and/or the medical criticality of the data (more critical findings over less critical findings).

At step S50, the compartments CP identified at step S20 and the supplementary information SI are provided to the text generation function LLM and the text generation function LLM is induced to generate a text summary TS per the identified compartment CP. Depending on the setup of the text generation function LLM, orders for the text generation function LLM may be formulated as prompts.

The contents of text summaries TS are not based on actual image data of the medical image data set MIDS, but rather on supplementary information SI outside of the medical image data set MIDS. The image data of a medical image data set MIDS is, by virtue of the compartments shown, only used as a pointer for what to look for in the (typically vast) supplementary information SI.

At step S60, the thus generated text summaries TS may be provided. This may involve showing the text summaries TS in the user interface 10, e.g., in a suitable graphical user interface. According to some examples, text summaries TS may be shown as an overlay over a representation R rendered from the medical image data set MIDS. Further, individual text summaries TS may be shown on a request base, i.e., reactive to a corresponding request by the user U. For instance, a text summary TS may be automatically shown if the user U hovers over a compartment CP in the representation R with a mouse cursor or the like.

At optional step S70, a holistic text summary may be generated. The holistic text summary may be configured so as to summarize individual compartment-specific text summaries TS. With that, the main findings relevant for a medical image data set MIDS can be concentrated in one summary. The holistic text summary may be generated by applying the text generation function LLM (or a further text generation function) to the individual text summaries TS. For deciding which aspects are relevant enough to be adopted for the holistic summary, in principle the same prioritization concepts as discussed in connection with steps S40 and S50 may be relied upon. According to some examples the holistic text summary may be generated based on a request from the user U.

At optional step S80, the text summaries TS may be classified according to at least two relevance levels. In other words, a classification result for each of the plurality of compartments CP based on the text summaries TS may be provided which indicates how pertinent the previously gathered information is for the respective compartment CP.

According to some examples, there may be only two relevance levels which may be: no findings (or "normal") and findings (or "not-normal"). With that, the user U may get a swift overview for which compartments CP there is no previous finding on record in the supplementary information.

Further, there may be additional relevance levels or even a continuum of relevance levels indicating how critical findings are from a medical perspective. For instance, a healed fracture may be attributed a lower criticality level as the classification result as compared to a previously reported lung nodule.

At optional step S81, the classification result may be provided. According to some examples, the text summaries TS displayed in the user interface 10 may be color coded according to the classification result and/or provided with a visual marker indicating the classification result. Moreover, the classification result may also be provided in a way that only text summaries TS of/above a certain relevance level are automatically provided to the user U and, therewith, brought to the user's attention.

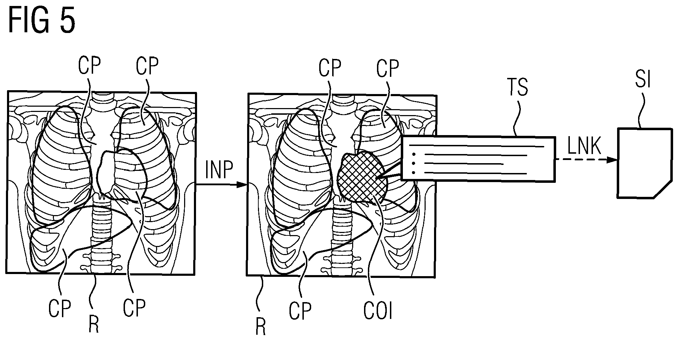

Figure 4 depicts optional method steps in connection with the provision of a text summary TS. Corresponding data streams are illustrated in Figure 5. The method comprises several steps. The order of the steps does not necessarily correspond to the numbering of the steps but may also vary between different embodiments of the present invention. Further, individual steps or a sequence of steps may be repeated.

At optional step S61, a compartment of interest COI is obtained, for which a text summary TS is deliberately being generated and/or provided to the user U. According to some embodiments, the step of obtaining a compartment of interest COI is part of step S60, i.e., carried out for deciding which one(s) of the generated text summaries TS are actually provided to the user U. According to other embodiments, step S61 may also carried out independently of the step S60.

According to some examples, the compartment of interest COI may be determined based on interactions of the user U with the medial image data set MIDS. An example for such like process is described in the following in connection with optional steps D10-D30.

At step D10, a representation R of the medical image data set MIDS is rendered. The representation R may relate to the slice of the medical image data set MIDS the user U is currently reviewing. Further, the representation R may be a rendering of a three-dimensional view of the medical image data set MIDS. The rendering of the representation R may be based on one or more imaging parameters set by the user U, such as viewing angles, filters, contrast and image enhancement settings.

At step D20, the representation R is displayed to the user U, for instance in a graphical user interface running in user interface 10.

Next, at optional step D30, a user input INP with respect to the representation R is received and analyzed if it is directed to a particular compartment CP within the medical image data set MIDS. This particular compartment may then be identified as the compartment of interest COI. For instance, the user input INP may comprise a moving an input device such as a mouse cursor to a compartment CP shown in the representation R, an activation of a measurement tool specific for a compartment CP, clicking on a compartment CP shown in the representation R, and the like.

At optional step S62, the text summary TS corresponding to the compartment of interest COI may be selected from the available text summaries TS as generated in step S50. As an alternative, for cases in which no pre-generated text summaries TS are available, the text summary TS may be deliberately requested for the compartment of interest COI.

That followed, the text summary TS of the compartment of interest COI may be provided. This may be done in roughly the same way as in described in connection with step S60.

As mentioned, the text summaries TS are intended to have concise format focused on the most valuable information for the user U. However, if the user U wants to get more information for a particular compartment, the user U may request an expanded version of the text summary TS according to an optional modification.

Specifically, after the receipt of a corresponding request to expand a certain text summary TS (optional step D40), the text generation function LLM may be used to generate an expanded version of original text summary TS. For instance, the expanded version may comprise further information from the supplementary information SI which was not considered relevant enough for the initial text summary TS. Further, the expanded text summary may comprise additional explanations and/or background information relating to those pieces of information already comprised in the initial text summary TS.

As another optional extension of the workflow, it may be enabled that the user U may input natural language queries which are then answered based on the text summaries TS and/or the supplementary information SI or even general information not specifically related to the patient.

Such queries may be received at optional step D50. They may be directly related to the text summaries TS (e.g., "only show information added to the file after the last radiological exam", "only show text summaries related to disease XY", etc.). Additionally, or as an alternative, queries may also more generally relate to the supplementary information SI (e.g., "when was the last blood test", "is the patient a smoker", etc.) or more general aspects (e.g., "which kind of examination is suggested to safeguard the diagnosis of disease XY according to our inhouse guidelines").

At optional step S65 an answer to those queries is obtained. Thereby the natural language understanding capabilities of text generation functions LLM may be leveraged to first understand the query and, secondly, translate the query to machine-understandable instructions to adapt the text summaries TS or look for additional information.

One issue with text generation functions LLM and, in particular, language models is that it is difficult for a user U to verify the correctness of the information provided. Optional steps S66 and S67 are directed to support the user U in this regard.

Specifically, it is proposed to obtain a link LNK to a source for an item or piece of information in the text summary TS. The source may be the data element in the supplementary information SI the corresponding information in the text summary TS was extracted from. As the case may be, the source may be a document such as a medical report, a referral letter, a protocol of a patient interview, an anamnesis questionnaire or the like.

The link LNK may be an electronic link to that item of the supplementary information SI with which the item can be directly retrieved from the medical information system 40.

At optional step S67, the link LNK is provided. According to some examples, the link LNK may be directly included in the text summary TS. Upon displaying the text summary TS, the link LNK may be visualized as an action field or button in the text summary TS. Upon activating the link LNK, the source document may be retrieved and shown to the user U. According to some examples, this may include automatically highlighting those passages in the source document relating to the corresponding piece of information in the text summary TS.

Another optional extension deals with an automated change assessment based on the generated text summaries TS in optional step S68. For instance, changes may be systematically assessed by time filtering the supplementary information SI. With that, more recent developments may be contrasted with the status quo at an earlier point in time. This enables, to bring recent changes to the attention of the user U.

In Figure 6, a schematic representation of the text generation function LLM according to an embodiment is shown. The text generation function LLM according to this embodiment comprises a transformer architecture. The transformer architecture follows an encoder-decoder structure and comprises an encoder ENC and a decoder DEC. In brief, the task of the encoder ENC is to map an input INPT to a sequence of continuous representations, which is then fed into a decoder DEC. The decoder DEC receives the output of the encoder ENC together with the decoder output OUTR at a previous iteration to generate an output OUT. Specifically, the input INPT may be the supplementary information SI and an indication of a compartment CP for which the text summary TS is to be generated. The output OUT is the text summary TS for that compartment CP based on the supplementary information SI.

The encoder ENC of this embodiment may comprise of a stack of N=6 identical layers. For the sake of easy reference, only one layer xN is shown in the drawing. Further, N may also be set to different values and, in particular, to values greater than N=6 according to the respective task. Each layer xN of the encoder ENC comprises two sublayers L1 and L3. The first sublayer L1 implements a so-called multi-head self-attention mechanism. Specifically, the first sublayer L1 may be configured to determine how relevant a particular word is with regard to other words in the input INPT. This may be represented as an attention vector. With that, it may be decided, if a certain passage in the supplementary information is related to compartment CP for which the text summary TS is to be generated. To avoid any bias, multiple attention vectors per word may be generated and fed into a weighted average to compute the final attention vector of every word. The second sublayer L3 is a fully connected feed-forward network which may, for example, comprise two linear transformations with Rectified Linear Unit (ReLU) activation in between. The N=6 layers of the encoder ENC apply the same linear transformations to all the words in the input INPT, but each layer employs different weight and bias parameters to do so. Each sublayer L1, L3 is succeeded by a normalization layer L2, which normalizes the sum computed between the input fed into the respective sublayer L1, L3, and the output generated by the respective sublayer L1, L3 itself. In order to capture information about the relative positions of the words in the input INPT, positional encodings PE are generated based on input embeddings INPT-E prior to being fed into the layers xN. The positional encodings PE are of the same dimension as the input embeddings INPT-E and may be generated using sine and cosine functions of different frequencies. Then, the positional encodings PE may be simply summed to the input embeddings INPT-E in order to inject the positional information PE. Input embeddings INPT-E may be, as usual, a representation of each word in the input INPT, typically in the form of a real-valued vector that encodes the meaning of the word such that the words that are closer in the vector space are expected to be similar in meaning. According to some examples, a neural network may be used to generate the input embeddings INPT-E.

The decoder DEC of this embodiment may also comprise of a stack of N=6 identical layers xN each comprising three sublayers L4, L1, L3 which may be succeeded by a normalization layer L2 as explained in connection with the encoder ENC. For the sake of easy reference, only one layer xN of the decoder DEC is shown in the drawing. Further, N may also be set differently and, in particular, greater than N=6 according to the respective task. While the sublayers L1 and L3 of the decoder DEC correspond in their functionality to the respective sublayers L1 and L3 of the encoder ENC, sublayer L4 receives the previous output OUTR of the decoder DEC (optionally transformed into corresponding embeddings and augmented with positional information if the output is a sequence of words), and implements multi-head self-attention over it weighing how important individual elements of the previous output vector OUTR are. That followed, the values from the first sublayer L4 of the decoder DEC are input in the L1-sublayer of the decoder DEC. This sublayer L1 of the de-coder DEC implements a multi-head self-attention mechanism similar to the one implemented in the first sublayer L1 of the encoder ENC. On the decoder side, this multi-head mechanism receives the values from the previous decoder sublayer L4 and the output of the encoder ENC. This allows the decoder to attend to all the words in parallel. Like in encoder ENC part, the output of the L1 sublayers is passed into a feed-forward layer L2, which will make the output vectors form into something which is easily acceptable by another decoder block or a linear layer. After all layers xN of the decoder DEC have been processed, the intermediate result is fed into a linear layer L5 which may be another feed-forward layer. It is used to expand the dimensions into a format expected for computing the output vector OUT, in this case a pre-cursor for the text summary TS. That followed, the result is passed through a Softmax Layer L6, which transforms the result into the text summary TS.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention. Independent of the grammatical term usage, individuals with male, female or other gender identities are included within the term.

## Claims

1. Computer-implemented method for providing a text summary (TS) for a medical image data set (MIDS) comprising the following steps:
- receiving (S10) the medical image data set (MIDS) of a patient,
- identifying (S20) at least one compartment (ORG) in the medical image data set (MIDS),
- accessing (S30) supplementary information (SI) associated to the patient in a medical information system (40),
- providing (S40) a text generation function (LLM) configured to provide, for a specific compartment (ORG), a natural language text summary (TS) summarizing medical information pertaining to the specific compartment,
- applying (S50) the text generation function (LLM) to the supplementary information (SI) so as to generate a text summary (TS) for the at least one compartment (ORG), and
- providing (S60) the text summary (TS) to a user (U) via a user interface (10).

2. Method according to claim 1, wherein
the text generation function (LLM), comprises a transformer network and/or a large language model.

3. Method according any one of the preceding claims, wherein
- the step of identifying (S20) comprises identifying a plurality of different compartments (ORG) in the medical image data set (MIDS), and
- the step of applying (S40) comprises applying (S50) the text generation function (LLM) to the supplementary information (SI) so as to generate a respective text summary (TS) for each of the plurality of compartments (ORG).

4. Method according to claim 3, further comprising:
- receiving (S71), from the user (U), a request for a holistic text summary (H-TS) for the medical image data set (MIDS),
- generating (S70) a holistic text summary (H-TS) by applying the text generation function (LLM) to the respective text summaries (TS), wherein the holistic text summary (H-TS) comprises a synopsis of the respective text summaries (TS).

5. Method according to any one of claims 3 or 4, further comprising
- classifying (S80) the plurality of different compartments based on respective text summaries (TS) according to at least two relevance levels so as to provide a classification result for each of the plurality of compartments (ORG), optionally wherein one of the relevance levels indicates the absence of any medically relevant finding in the corresponding compartment (ORG),
- providing (S81) the classification results to the user (U) via the user interface (10).

6. Method according to any one of claims 3 to 5, wherein the step of providing (S60) comprises:
- obtaining (S61) a compartment of interest (COI) of the user (U),
- selecting (S62) the text summary (TS) pertaining to the compartment of interest (COI) from the respective text summaries (TS), and
- providing (S63) the selected text summary (TS) to the user (U) via the user interface (10).

7. Method according to claim 6, wherein
the step of obtaining (S61) the compartment of interest (COI) comprises:
- receiving (D30) a user input (INP) into the user interface (10), and
- determining the compartment of interest (COI) based on the user input (INP).

8. Method according to claim 7, further comprising
- generating (D10) a representation (R) of the medical image data set (MIDS) for displaying to the user (U) via the user interface (10),
- displaying (D20) the representation (R) to the user (U) via the user interface (10), wherein
- in the step of receiving (D30) the user input (INP), the user input (INP) is directed to the representation (R).

9. Method according to any one of the preceding claims, wherein
- the text summary (TS) has a predetermined text length, and
- the step of providing (S60) comprises
- receiving (D40) a request (REQ) from the user (U) to provide additional details,
- expanding (S64) the text summary (TS) into a text of a text length longer than the predetermined text length based on the supplementary information (SI) using the text generation function (LLM).

10. Method according to any of the preceding claims, further comprising
- receiving (D50) a natural language query from the user (U) regarding the text summary (TS),
- inputting (S65) the natural language query into the text generation function (LLM) so as to generate a natural language answer to the natural language query by applying the text generation function (LLM) to the supplementary information (SI),
- outputting the natural language answer via the user interface (10).

11. Method according to any one of the preceding claims, wherein the step of providing (S60) the text summary (TS) comprises
- obtaining (S66), for at least one item in the text summary (TS), an electronic link (LNK) to the source of the item in the supplementary information (SI), wherein the link (LNK) is configured to enable retrieving the source in the user interface (10).
- including (S67) the link (LNK) in the text summary (TS)

12. Method according to any one of the preceding claims, wherein
- the text generation function (LLM) is further configured to determine a change of the text summary (TS) as function of data status of the supplementary information (SI) over time,
and
- the step of providing the text summary (TS) comprises indicating (S68) the change for the user (U) in the user interface (10).

13. System (1) for providing a text summary (TS) for a medical image data set (MIDS) comprising an interface unit and a computing unit, wherein
- the interface unit is configured to:
- receive (S10) the medical image data set (MIDS) of a patient,
- access (S30) supplementary information (SI) associated to the patient in a medical information system (40), and
- provide (S60) the text summary (TS) to a user (U), and
- the computing unit is configured to:
- identify (S20) at least one compartment (ORG) in the medical image data set (MIDS),
- provide (S40) a text generation function (LLM) configured to provide, for a specific compartment, a natural language text summary (TS) summarizing medical information pertaining to the specific compartment, and
- apply (S50) the text generation function (LLM) to the supplementary information (SI) so as to generate a text summary (TS) for the at least one compartment (ORG).

14. Computer program product comprising program elements which induce a computing unit (20) of a system (1) for providing a text summary (TS) for a medical image data set (MIDS) to perform steps of the method according to any of claims 1 to 12 when the program elements are loaded into a memory of the computing unit (20).

15. Computer-readable medium on which program elements are stored that are readable and executable by a computing unit (20) of a system (1) for providing a text summary (TS) for a medical image data set (MIDS) to perform steps of the method according to any of claims 1 to 12 when the program elements are executed by the computing unit (20).
